# EUROPEAN PATENT SPECIFICATION

(11) **EP 1 861 169 B1**
(45) Date of publication and mention of the grant of the patent: **11.05.2016**
(21) Application number: 06730328.9
(22) Date of filing: 22.03.2006
(51) Int. Cl.: A61P 1/04, A61P 35/00, A61K 31/557, C07C 405/00

(54) **Use of 13,14-dihydro-15-keto-16,16-difluoro prostaglandin E1 or 13,14-dihydro-15-keto-16,16-difluoro-18-methyl prostaglandin E1 for the treatment of cancer**
Verwendung von 13,14-Dihydro-15-keto-16,16-difluoroprostaglandin E1 oder 13,14-dihydro-15-keto-16,16-difluoro-18-methyl prostaglandin E1 zur Behandlung von Krebs
Utilisation de la 13, 14-dihydro-15-céto-16,16-difluoro prostaglandine E1 ou de la 13,14-dihydro-15-céto-16,16-difluoro-18-méthyl prostaglandine E1 pour le traitement du cancer

(30) Priority: 21.03.2005 US 663200 P; 11.05.2005 US 679920 P; 30.09.2005 US 721976 P
(43) Date of publication of application: 05.12.2007
(60) Divisional application: 11170921.8
(73) Proprietor: Sucampo AG, 6300 Zug (CH); NORTH CAROLINA STATE UNIVERSITY, Raleigh, NC 27606 (US)
(72) Inventor: UENO, Ryuji, Maryland 21601 (US); KUNO, Sachiko, Maryland 21601 (US); BLIKSLAGER, Anthony, T., Apex, NC 27539 (US); MOESER, Adam, J., Raleigh, NC 27606 (US)
(74) Representative: Adam, Holger
(86) International application number: PCT/JP2006/306380
(87) International publication number: WO 2006/101244

(56) References cited:
- EP-A- 0 097 023
- EP-A- 0 098 141
- WO-A-2004/060377
- WO-A-2006/047476
- FR-A- 2 608 924
- US-A- 4 158 062
- US-A1- 2005 164 992
- US-A1- 2005 228 185
- US-B1- 6 265 440
- US-B1- 6 265 440
- US-B1- 6 492 417
- KABASHIMA K ET AL: "The prostaglandin receptor EP4 suppresses colitis, mucosal damage and CD4 cell activation in the gut" JOURNAL OF CLINICAL INVESTIGATION, NEW YORK, NY, US, vol. 109, no. 7, 2002, pages 883-893, XP002370491 ISSN: 0021-9738
- PLANCHON P ET AL: "Evidence for separate mechanisms of antiproliferative action of indomethacin and prostaglandin on MCF-7 breast cancer cells" LIFE SCIENCES, vol. 57, no. 12, 1995, pages 1233-1240, XP002397704 ISSN: 0024-3205
- KATO T ET AL: "ANTITUMOR ACTIVITY OF DELTA-7 PROSTAGLANDIN A-1 AND DELTA-12 PROSTAGLANDIN J-2 IN-VITRO AND IN-VIVO" CANCER RESEARCH, vol. 46, no. 7, 1986, pages 3538-3542, XP009071935 ISSN: 0008-5472
- CUPPOLETTI J; MALINOWSKA D H; CHAKRABARTI J; UENO R: "Effects of lubiprostone on human uterine smooth muscle cells", PROSTAGLANDINS AND OTHER LIPID MEDIATORS, vol. 86, no. 1-4 , - 1 June 2008 (2008-06-01), pages 56-60, XP022649881,
- NILIUS B; DROOGMANS G: "Amazing chloride channels: An overview", ACTA PHYSIOLOGICA SCANDINAVICA, vol. 117, no. 2, 1 February 2003 (2003-02-01), pages 119-147, GB
- OLSEN M. L ET AL.: "Expression of voltage-geted chloride channels in human glioma cells", THE JOURNAL OF NEUROSCIENCE, vol. 23, no. 13, 2 July 2003 (2003-07-02), pages 5572-5582,
- WINPENNY J P: "Lubiprostone Sucampo Pharmaceuticals/Takeda Pharmaceutical", IDRUGS, vol. 8, no. 5, May 2005 (2005-05), pages 416-422, XP008055564, gb
- LITTLE DIANNE; DEAN REBECCA A; YOUNG KAREN M; MCKANE SHAUN A; MARTIN LINDA D; JONES SAMUEL L; BLIKSLAGER ANTHONY T: "PI3K signaling is required for prostaglandin-induced mucosal recovery in ischemia-injured porcine ileum.", AMERICAN JOURNAL OF PHYSIOLOGY. GASTROINTESTINAL AND LIVER PHYSIOLOGY, vol. 284, no. 1, January 2003 (2003-01), pages G46-G56, usa
- BLIKSLAGER A T; ROBERTS M C; ARGENZIO R A: "Prostaglandin-induced recovery of barrier function in porcine ileum is triggered by chloride secretion", AMERICAN JOURNAL OF PHYSIOLOGY - GASTROINTESTINAL AND LIVER PHYSIOLOGY, vol. 276, no. 1, January 1999 (1999-01), pages G28-G36,

## Description

### TECHNICAL FIELD

The present invention relates to a compound and composition for use in treating a condition associated with reduced mucosal barrier function in a mammalian subject, namely cancer or premalignant condition.

### BACKGROUND ART

Epithelial tissues act as barriers between two fluid compartments, and the epithelial barrier function is provided by the epithelial cells and the tight junctions (hereinafter TJ or TJs) that connect them. TJs are the most apical components of the cell-cell junctional complexes, play a crucial role in the establishment and maintenance of cell polarity within tissues, and function as selective barriers to macromolecules and prevent diffusion of lipids and proteins between apical and basolateral membrane domain. TJs also create the variable barrier regulating paracelluler movement of molecules through epithelial sheet, thereby maintaining tissue homeostasis. Mucosal epithelial TJs are dynamic structures and subject to modulation during epithelial tissue remodeling, wound repair, inflammation and transformation into tumors. The association of abnormal TJ function and epithelial tumor development has been suggested by earlier studies showing alterations in the TJ structures of epithelial cancers.

There are a lot of reports on the crucial relationship between decrease or loss of mucosal TJ function and a number of cancers.

It is reported that Helicobacter pylori, which plays a role in the development of gastric carcinoma, disrupt the epithelial barrier function (Infection and Immunity 66(6): 2943-2950, 1998 and Science 300: 1430-1434, 2003). It is also reported that the down-regulation of the tight junction protein are common in advanced gastric cancer (Oncology Reports 13: 193-199, 2005).

It is reported that increased TJ permeability of the colon epithelium and consequently a decrease in epithelial barrier function precede the development of colon cancer (Carcinogenesis 20(8): 1425-1431, 1999).

It is reported that alterations in TJ function may be important in the development of both inflammatory disease of the urinary bladder and transitional cell carcinoma (International Journal of Molecular Medicine 16:3-9, 2005).

It is reported that loss of TJ function and TJ molecule expression are observed in human breast cancer (American Journal of Pathology 153(6):1767-1773, 1998 and Journal of Mammary Gland Biology and Neoplasia 8(4): 449-462, 2003).

It is reported that loss of tight junctions has a close relationship with structural atypia in the progression of human endometrial carcinomas and their malignant potential (Human Pathology 35(2), 159-164: 2004).

It is reported that the disruption of TJs, which is thought to contribute to epithelial tumorigenesis, was observed in ovarian cancer cells (The Journal of Biological Chemistry 280(28): 26233-26240, 2005).

It is reported that the alterations of the TJs were found in the oncocyctic tumors of the thyroid (Ultrastructural Pathology 22(6): 413-420, 1998).

It is reported that there is disorganization of tight junctions in hepatocellular carcinoma, and these structural anomalies may alter permeability barriers and limit intercellular communication, which could contribute to the proliferative behavior of the neoplastic cells (J. Submicrosc. Cytol. 15(3); 799-810, 1983).

Barrett's esophagus (BE) represents the most serious histological consequence of gastroesophageal reflux disease (GERD) that develops in 5-10% of patients with GERD. Barrett's esophagus is recognized as a premalignant condition, with the incidence of adenocarcinoma in those with Barrett's being much higher than in the general population.

It is reported that the paracellular barrier is distinctly different in Barrett's epithelium, and dramatic functional difference exists in barrier properties in Barrett's epithelium compared with normal esophagus (American Journal of Gastroenterology 98(8): 1901-1903, 2003).

It is reported that the alterations in TJ proteins in reflux esophagitis (GERD) most likely increase the permeability of the esophageal epithelium, thereby impairing the defense mechanism of this epithelium (Journal of Gastroenterology 40, 781-790, 2005).

Intestinal barrier function refers to the ability of the intestinal mucosa to prevent potentially harmful luminal components such as bacteria and associated toxins from transmigrating across the epithelium and gaining access to the systemic tissues. Breakdown of intestinal barrier function can result from a variety of pathologic conditions including ischemic injury, shock, stress, infectious diseases, and inflammatory bowel diseases (IBD).

Inflammatory bowel diseases (IBD) are defined by chronic, relapsing intestinal inflammation of obscure origin, of which the two major forms are Crohn's disease and ulcerative colitis. Both diseases appear to involve either a dysregulated immune response to gastrointestinal (GI) tract antigens, a breach in mucosal barrier function, and/or an adverse inflammatory reaction to a persistent intestinal infection, collagen disease, radiation therapy, orally administered medication, and the like.

Crohn's disease is characterized by thickened areas of the gastro-intestinal wall, with inflammation extending through all layers, deep ulceration and fissuring of the mucosa, and the presence of granulomas. Affected areas may occur in any part of the gastro-intestinal tract, although the terminal ileum is frequently involved, and they may be interspersed with areas of relatively normal tissue. In ulcerative colitis, disease is also present within the colon and rectum. Inflammation is superficial but continuous over the affected area and granulomas are rare.

It is also becoming increasingly evident that many critically ill patients suffer from multiple organ failure initiated by poor splanchnic perfusion. Multiple organ failure is the leading cause of death in intensive care unit patients.

These gastrointestinal mucosal disorders are generally difficult to cure and, in some cases, surgical treatments are applied thereto. Currently available medicinal therapies for these disorders include steroids, Salazopyrin (general name is Salicylazosulfapyridine), immunosuppressive agents, etc. However, the steroidal drugs show side effects when administered in large dosages over a long time period, and the immunosuppressive agents must be carefully used because of the very harmful side effects. Hence, it is desired to develop a medicine which is effective to the treatment of intractable gastrointestinal mucosal disorders and which can be used safely over a long time period.

Prostaglandins (hereinafter, referred to as PGs) are members of class of organic carboxylic acids, which are contained in tissues or organs of humans or other mammals, and exhibit a wide range of physiological activity. PGs found in nature (primary PGs) generally have a prostanoic acid skeleton as shown in the formula (A):

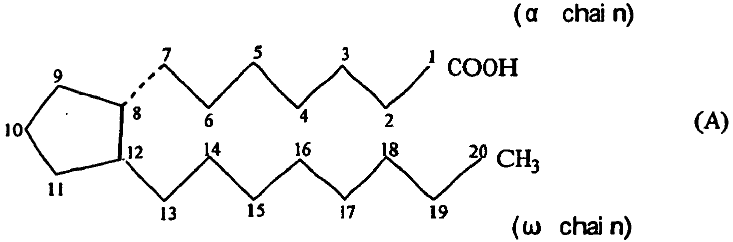

PGs are classified into several types according to the structure and substituents on the five-membered ring, for example,
Prostaglandins of the A series (PGAs); 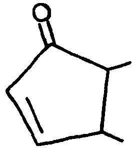
Prostaglandins of the B series (PGBs); 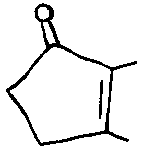
Prostaglandins of the C series (PGCs) ; 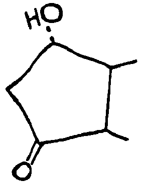
Prostaglandins of the D series (PGDs) ; 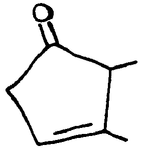
Prostaglandins of the E series (PGEs); 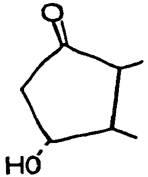
Prostaglandins of the F series (PGFs); 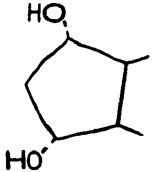
and the like. Further, they are classified into PG₁s containing a 13,14-double bond; PG₂s containing 5,6- and 13,14-double bonds; and PG₃s containing 5,6-, 13,14- and 17,18-double bonds. PGs are known to have various pharmacological and physiological activities, for example, vasodilatation, induction of inflammation, platelet aggregation, stimulating uterine muscle, stimulating intestinal muscular activity, anti-ulcer effects and the like. The major prostaglandins produced in the human gastrointestinal (GI) system are those of the E, I and F series (Sellin, Gastrointestinal and Liver Disease: Pathophysiology, Diagnosis, and Management. (WB Saunders Company, 1998); Robert, Physiology of the Gastrointestinal Tract 1407-1434 (Raven, 1981); Rampton, Prostaglandins: Biology and Chemistry of Prostaglandins and Related Eicosanoids 323-344 (Churchill Livingstone, 1988); Hawkey, et al., Gastroenterology, 89: 1162-1188 (1985); Eberhart, et al., Gastroenterology, 109: 285-301 (1995)).

Under normal physiological conditions, endogenously produced prostaglandins play a major role in maintaining GI function, including regulation of intestinal motility and transit, and regulation of fecal consistency. (Sellin, Gastrointestinal and Liver Disease: Pathophysiology, Diagnosis, and Management. (WB Saunders Company, 1998); Robert, Physiology of the Gastrointestinal Tract 1407-1434 (Raven, 1981);Rampton, Prostaglandins: Biology and Chemistry of Prostaglandins and Related Eicosanoids 323-344 (Churchill Livingstone, 1988); Hawkey, et al., Gastroenterology, 89: 1162 -1188 (1985); Eberhart, et al. , Gastroenterology, 109: 285-301 (1995); Robert, Adv Prostaglandin Thromboxane Res, 2: 507-520 (1976); Main, et al., Postgrad Med J, 64 Suppl 1: 3-6 (1988) ; Sanders, Am J Physiol, 247: G117 (1984); Pairet, et al., Am J Physiol. , 250 (3 pt 1) : G302-G308 (1986); Gaginella, Textbook of Secretory Diarrhea 15-30 (Raven Press, 1990)). When administered in pharmacological doses, both PGE₂ and PGF_{2α} have been shown to stimulate intestinal transit and to cause diarrhea (Robert, Physiology of the Gastrointestinal Tract 1407-1434 (Raven, 1981); Rampton, Prostaglandins: Biology and Chemistry of Prostaglandins and Related Eicosanoids 323-344 (Churchill Livingstone, 1988); Robert, Adv Prostaglandin Thromboxane Res, 2:507-520 (1976)). Furthermore, the most commonly reported side effect of misoprostol, a PGE₁ analogue developed for the treatment of peptic ulcer disease, is diarrhea (Monk, et al., Drugs 33 (1): 1-30 (1997)).

PGE or PGF can stimulate intestinal contraction, but the enteropooling effect is poor.

Accordingly, it is impractical to use PGEs or PGFs as cathartics because of side effects such as intestinal contraction that cause abdominal pain.

Multiple mechanisms, including modifying enteric nerve responses, altering smooth muscle contraction, stimulating mucous secretion, stimulating cellular ionic secretion (in particular electrogenic CI⁻ transport) and increasing intestinal fluid volume have been reported to contribute to the GI effects of prostaglandins (Robert, Physiology of the Gastrointestinal Tract 1407-1434 (Raven, 1981); Rampton, Prostaglandins: Biology and Chemistry of Prostaglandins and Related Eicosanoids 323-344 (Churchill Livingstone, 1988); Hawkey, et al., Gastroenterology, 89: 1162-1188 (1985); Eberhart, et al., Gastroenterology, 109: 285-301 (1995); Robert, Adv Prostaglandin Thromboxane Res, 2:507-520 (1976); Main, et al., Postgrad Med J, 64 Suppl 1: 3-6 (1988); Sanders, Am J Physiol, 247: G117 (1984) ; Pairet, et al., Am J Physiol, 250 (3 pt 1) : G302-G308 (1986); Gaginella, Textbook of Secretory Diarrhea 15-30 (Raven Press, 1990); Federal Register Vol. 50, No. 10 (GPO,1985) ; Pierce, et al., Gastroenterology 60 (1): 22-32 (1971); Beubler, et al., Gastroenterology, 90: 1972 (1986); Clarke, et al., Am J Physiol 259: G62 (1990); Hunt, et al., J Vet Pharmacol Ther, 8 (2): 165-173 (1985); Dajani, et al., Eur J Pharmacol, 34(1): 105-113 (1975); Sellin, Gastrointestinal and Liver Disease: Pathophysiology, Diagnosis, and Management 1451-1471 (WB Saunders Company, 1998)). Prostaglandins have additionally been shown to have cytoprotective effects (Sellin, Gastrointestinal and Liver Disease: Pathophysiology, Diagnosis, and Management. (WB Saunders Company, 1998); Robert, Physiology of the Gastrointestinal Tract 1407-1434 (Raven, 1981); Robert, Adv Prostaglandin Thromboxane Res 2:507-520 (1976); Wallace, et al., Aiiment Pharmacol Ther 9: 227-235 (1995)).

U. S. Patent No. 5,225,439, 5, 166, 174, 5,284,858, 5,428,062, 5,380,709, 5,876,034 and 6,265,440 describe that certain prostaglandin E compounds are effective for the treatment of ulcers such as duodenal ulcer and gastric ulcer.

U.S. Patent No. 5,317,032 to Ueno et al. describes prostaglandin compound cathartics, including the existence of bicyclic tautomers and U.S. Patent No. 6,414,016 to Ueno describes the bicyclic tautomers as having pronounced activity as anti-constipation agents. The bicyclic tautomers, substituted by one or more halogen atoms can be employed in small doses for relieving constipation. At the C-16 position, especially, fluorine atoms, can be employed in small doses for relieving constipation.

U.S. Patent application publication Nos. 2003/0130352 and 2003/0166632 to Ueno et al. describes a prostaglandin compound that opens and activates chloride channels, especially ClC channels, particularly the ClC-2 channel.

U.S. Patent application publication No.2003/0119898 to Ueno et al. describes a specific composition of a halogenated prostaglandin compound for the treatment and prevention of constipation.

The inventors have previously demonstrated in a porcine in vitro model of intestinal ischemia that repair of intestinal barrier function is mediated through a mechanism which involves prostaglandin (PG) production through cyclooxygenase-dependent pathways and activated Cl⁻ secretion (Am J Physiol, 276: G28-36, 1999 and Am J Physiol Gastrointest Liver Physiol, 284:G46-56, 2003).

The inventors have also previously demonstrated that PGE₂ and PGI₂ have a synergistic role in restoration of intestinal barrier function, whereas the addition of each alone had a diminished effect (J. Clin. Invest. 1997. 100(8):1928-1933).

In addition, it is indicated that PGE₁-stimulated Cl⁻ secretion decreases in restoration of intestinal barrier function (J. Clin. Invest. 76:1828-1836,1985).

In further studies, Misoprostol was shown to have effects on barrier function, although deoxy-PGE1 and Sulprostone had not effects on it (Am. J. Physiol. Gastrointest. Liver Physiol. 281:G375-81, 2001).

Planchon P. at al, Life Sciences, vol 57 no.12,1995, pages 1233-1240, disclose the antiproliferative properties of PGE1, PGE2, PGD2 on MCF-7 breast cancer cells.

### DISCLOSURE OF THE INVENTION

An object of the present invention is to provide a compound and composition for use in treating cancer or premalignant condition in a mammalian subject, the cancer being, for example, selected from the group consisting of mammary carcinoma, colon cancer and pulmonary carcinoma.

In spite of the prior art, the inventors have found that a specific prostaglandin compound has a significant effect on a conformational change in the TJs that results in recovery of mucosal barrier function, which resulted in the completion of the present invention.

Namely, the present invention relates to the use of 13,14-dihydro-15-keto-16,16-difluoro prostaglandin E₁ or 13,14-dihydro-15-keto-16,16-difluoro-18-methyl-prostaglandin E₁.

A preferred embodiment of the use according to the present invention as described above is the subject-matter of dependent claim 2.

Further, the present invention relates to a composition for use in the treatment of cancer or premalignant condition, the cancer being, for example, selected from the group consisting of mammary carcinoma, colon cancer and pulmonary carcinoma in a mammalian subject, which comprises an effective amount of 13, 14-dihydro-15-keto-16,16-difluoro prostaglandin E₁ compound or 13,14-dihydro-15-keto-16,16-difluoro-18-methyl-prostaglandin E₁.

A preferred embodiment of the composition as described above is the subject-matter of dependent claim 4.

### BRIEF DESCRIPTION OF DRAWINGS

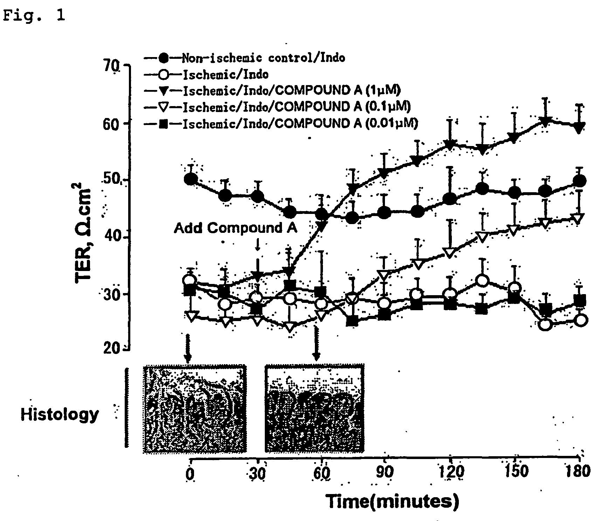

Fig. 1 is a graph showing tansepithelial electrical resistance (TER) in response to COMPOUND A (13,14-dihydro-15-keto-16,16-difluoro-PGE₁) in ischemia-injured porcine ileum. Values represent means ± SE; n=6. Ischemic tissues bathed in indomethacin (5×10⁻⁶ M) containing Ringer's solution demonstrated marked elevations in transepithelial electrical resistance (TER) in the presence of COMPOUND A (0.1 µM and 1 µM doses), added after a 30-min equilibration period.

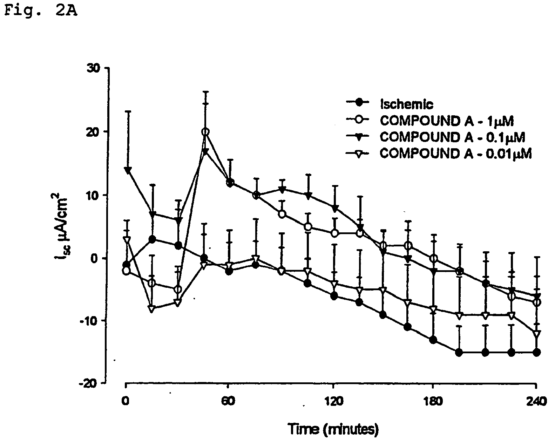

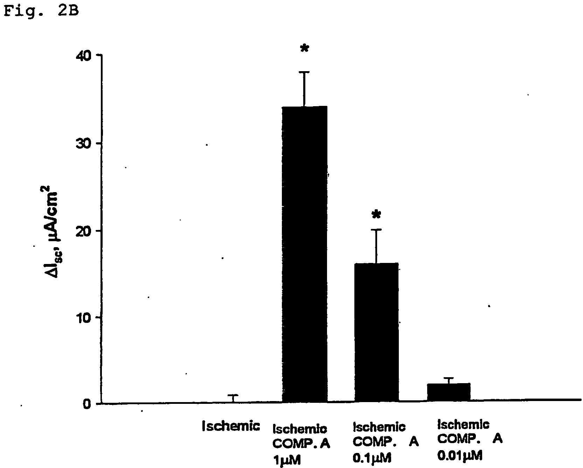

Fig. 2A is a graph showing the effect of COMPOUND A on short circuit current. Fig. 2B is a graph showing change in short circuit current (*ΔIsc*) in response to the COMPOUND A in ischemia-injured porcine ileum. In both figures, values represent means ± SE; n=6. Ischemic tissues were bathed in indomethacin (5×10⁻⁶ M) containing Ringer's solution. Significant (P < 0.05) increases in Cl⁻ secretion, indicated by both short circuit current *(Isc)* and the absolute change in short-circuit current (*ΔIsc*), were observed in response to the treatment with increasing doses of COMPOUND A (*P < 0.05).

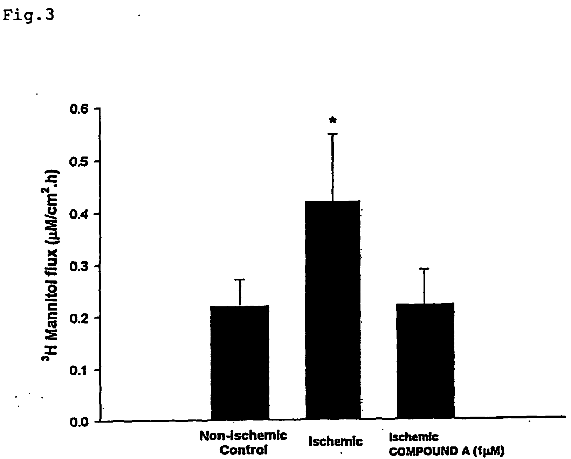

Fig.3 is a graph showing effect of COMPOUND A on mucosal-to-serosal ³H-Mannitol Fluxes. Values represent means ± SE, n=4. Ischemic tissues were bathed in indomethacin (5×10⁻⁶ M) containing Ringer's solution. Porcine ileum subjected to intestinal ischemia and exhibited increased ³H-mannitol mucosal-to-serosal fluxes compared with non-ischemic control. Application of 1µM COMPOUND A reduced ³H-mannitol mucosal-to-serosal fluxes to non-ischemic control levels. **P* < 0.05.

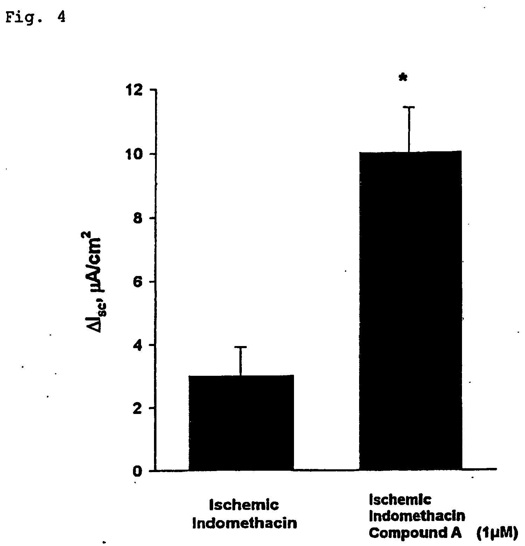

Fig.4 is a graph showing change in short circuit current in response to the treatment with COMPOUND A in ischemia-injured porcine ascending colon.
Fog. 5 is a graph showing a transepithelial electrical resistance (TER) in response to the treatment with COMPOUND A in ischemia-injured porcine ascending colon.

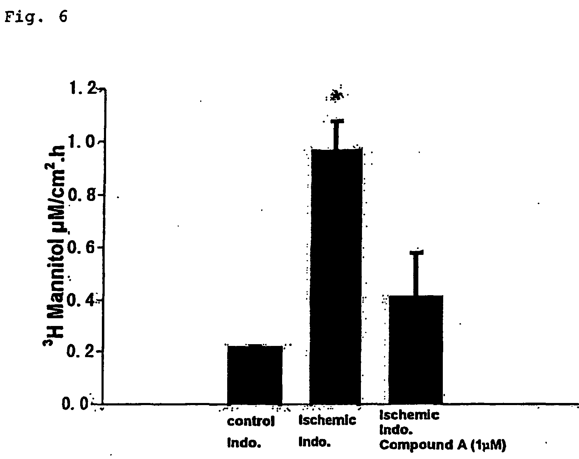

Fig.6 is a graph showing serosal to mucosal ³H-mannitol fluxes in response to the treatment of COMPOUND A in ischemia-injured porcine ascending colon.

### DETAILED DESCRIPTION OF THE INVENTION

The nomenclature of the prostaglandin compounds used herein is based on the numbering system of the prostanoic acid represented in the above formula (A).

The formula (A) shows a basic skeleton of the C-20 carbon atoms, but the present invention is not limited to those having the same number of carbon atoms. In the formula (A), the numbering of the carbon atoms which constitute the basic skeleton of the PG compounds starts at the carboxylic acid (numbered 1), and carbon atoms in the α-chain are numbered 2 to 7 towards the five-membered ring, those in the ring are 8 to 12, and those in the ω-chain are 13 to 20. When the number of carbon atoms is decreased in the α-chain, the number is deleted in the order starting from position 2; and when the number of carbon atoms is increased in the α-chain, compounds are named as substitution compounds having respective substituents at position 2 in place of the carboxy group (C-1). Similarly, when the number of carbon atoms is decreased in the ω-chain, the number is deleted in the order starting from position 20; and when the number of carbon atoms is increased in the ω-chain, the carbon atoms beyond position 20 are named as substituents. Stereochemistry of the compounds is the same as that of the above formula (A) unless otherwise specified.

In general, each of the terms PGD, PGE and PGF represents a PG compound having hydroxy groups at positions 9 and/or 11, but in the present specification, these terms also include those having substituents other than the hydroxy group at positions 9 and/or 11. Such compounds are referred to as 9-dehydroxy- 9-substituted-PG compounds or 11-dehydroxy-11-substituted-PG compounds. A PG compound having hydrogen in place of the hydroxy group is simply named as 9- or 11-deoxy-PG compound.

As stated above, the nomenclature of the PG compounds is based on the prostanoic acid skeleton. However, in case the compound has a similar partial structure as a prostaglandin, the abbreviation of "PG" may be used. Thus, a PG compound of which α-chain is extended by two carbon atoms, that is, having 9 carbon atoms in the α-chain is named as 2-decarboxy-2-(2-carboxyethyl)-PG compound. Similarly, a PG compound having 11 carbon atoms in the α-chain is named as 2-decarboxy-2-(4-carboxybutyl)-PG compound. Further, a PG compound of which ω-chain is extended by two carbon atoms, that is, having 10 carbon atoms in the ω-chain is named as 20-ethyl-PG compound. These compounds, however, may also be named according to the IUPAC nomenclatures.

Suitable "pharmaceutically acceptable salts" include conventionally used non-toxic salts, for example a salt with an inorganic base such as an alkali metal salt (such as sodium salt and potassium salt), an alkaline earth metal salt (such as calcium salt and magnesium salt), an ammonium salt; or a salt with an organic base, for example, an amine salt (such as methylamine salt, dimethylamine salt, cyclohexylamine salt, benzylamine salt, piperidine salt, ethylenediamine salt, ethanolamine salt, diethanolamine salt, triethanolamine salt, tris(hydroxymethylamino) ethane salt, monomethyl- monoethanolamine salt, procaine salt and caffeine salt), a basic amino acid salt (such as arginine salt and lysine salt), tetraalkyl ammonium salt and the like. These salts may be prepared by a conventional process, for example from the corresponding acid and base or by salt interchange.

In the present invention, the PG compound which is dihydro between 13 and 14, and keto(=O) at 15 position may be in the keto-hemiacetal equilibrium by formation of a hemiacetal between hydroxy at position 11 and keto at position 15.

For example, it has been revealed that with two fluorine atoms, the compound contains a tautomeric isomer, bicyclic compound.

If such tautomeric isomers as above are present, the proportion of both tautomeric isomers varies with the structure of the rest of the molecule or the kind of the substituent present. Sometimes one isomer may predominantly be present in comparison with the other. However, it is to be appreciated that the present invention includes both isomers.

Further, the 15-keto-PG compounds used in the invention include the bicyclic compound and analogs or derivatives thereof.

Furthermore, while the compounds used in the invention may be represented by a formula or name based on keto-type regardless of the presence or absence of the isomers, it is to be noted that such structure or name does not intend to exclude the hemiacetal type compound.

In the present invention, any of isomers such as the individual tautomeric isomers, the mixture thereof, or optical isomers, the mixture thereof, a racemic mixture, and other steric isomers may be used in the same purpose.

The compounds used in the present invention may be prepared by the method disclosed in USP Nos. 5, 073, 569, 5, 166, 174, 5, 221, 763, 5, 212, 324, 5, 739, 161 and 6,242,485.

A disorder selected from the group consisting of mammary carcinoma, colon cancer and pulmonary carcinoma in a mammalian subject may be treated by administering the above described prostaglandin compound to the subject. The subject may be any mammalian subject including a human. The compound may be applied systemically or topically. Usually, the compound may be administered by oral administration, intravenous injection (including infusion), subcutaneous injection, intra rectal administration, intra vaginal administration, transdermal administration and the like.

The dose may vary depending on the strain of the animal, age, body weight, symptom to be treated, desired therapeutic effect, administration route, term of treatment and the like. A satisfactory effect can be obtained by systemic administration 1-4 times per day or continuous administration at the amount of 0.001-1000µg, more preferably 0.01-100µg of active ingredient per one kg body weight per day.

The prostaglandin compound may preferably be formulated in a pharmaceutical composition suitable for administration in a conventional manner. The composition may be those suitable for oral administration, injection or perfusion as well as it may be an external agent, suppository or pessary.

The composition of the present invention may further contain physiologically acceptable additives. Said additives may include excipient, diluent, filler, resolvent, lubricant, adjuvant, binder, disintegrator, coating agent, cupsulating agent, ointment base, suppository base, aerozoling agent, emulsifier, dispersing agent, suspending agent, thickener, tonicity agent, buffering agent, soothing agent, preservative, antioxidant, corrigent, flavor, colorant, a functional material such as cyclodextrin and biodegradable polymer and stabilizer. The additives are well known to the art and may be selected from those described in general reference books of pharmaceutics.

The amount of the above-defined prostaglandin compound in the composition of the invention may vary depending on the formulation of the composition, and may generally be 0.000001-10.0%, more preferably 0.00001-5.0%, most preferably 0.0001-1%.

Examples of solid compositions for oral administration include tablets, troches, sublingual tablets, capsules, pills, powders, granules and the like. The solid composition may be prepared by mixing one or more active ingredients with at least one inactive diluent. The composition may further contain additives other than the inactive diluents, for example, a lubricant, a disintegrator and a stabilizer. Tablets and pills may be coated with an enteric or gastroenteric film, if necessary. They may be covered with two or more layers. They may also be incorporated in a sustained release material, or microcapsulated. Additionally, the compositions may be capsulated by means of an easily degradable material such gelatin. They may be further dissolved in an appropriate solvent such as fatty acid or its mono, di or triglyceride to provide a soft capsule. Sublingual tablet may be used in need of fast-acting property.

Examples of liquid compositions for oral administration include emulsions, solutions, suspensions, syrups and elixirs and the like. Said composition may further contain a conventionally used inactive diluents e.g. purified water or ethyl alcohol. The composition may contain additives other than the inactive diluents such as adjuvant e.g. wetting agents and suspending agents, sweeteners, flavors, fragrance and preservatives.

The composition of the present invention may be in the form of spraying composition, which contains one or more active ingredients and may be prepared according to a known method.

Examples of the injectable compositions of the present invention for parenteral administration include sterile aqueous or non-aqueous solutions, suspensions and emulsions. Diluents for the aqueous solution or suspension may include, for example, distilled water for injection, physiological saline and Ringer's solution.

Non-aqueous diluents for solution and suspension may include, for example, propylene glycol, polyethylene glycol, vegetable oils such as olive oil, alcohols such as ethanol and polysorbate. The composition may further comprise additives such as preservatives, wetting agents, emulsifying agents, dispersing agents and the like. They may be sterilized by filtration through, e.g. a bacteria-retaining filter, compounding with a sterilizer, or by means of gas or radioisotope irradiation sterilization. The injectable composition may also be provided as a sterilized powder composition to be dissolved in a sterilized solvent for injection before use.

The present external agent includes all the external preparations used in the fields of dermatology and otolaryngology, which includes ointment, cream, lotion and spray.

Another form of the present invention is suppository or pessary, which may be prepared by mixing active ingredients into a conventional base such as cacao butter that softens at body temperature, and nonionic surfactants having suitable softening temperatures may be used to improve absorbability.

The term "treatment" or "treating" used herein includes any means of control such as prevention, care, relief of the condition, attenuation of the condition and arrest of progression.

According to the present invention, the prostaglandin compound induces a conformational change in the tight junction that results in recovery of mucosal barrier function.

As is described above, there is the crucial relationship between decrease or loss of mucosal Tight junction function and a number of cancers, so that another aspect of the condition reduced mucosal barrier function includes cancer or premalignant condition.

The cancers used herein include, but are not limited to, esophageal carcinoma, gastric carcinoma, duodenal cancer, small intestinal cancer, appendiceal cancer, large bowel cancer, colon cancer, rectum cancer, anal carcinoma, pancreatic cancer, liver cancer, gallbladder cancer, spleen cancer, renal carcinoma, bladder cancer, prostatic carcinoma, testicular carcinoma, uterine cancer, ovarian cancer, mammary carcinoma, pulmonary carcinoma and thyroid carcinoma.

The pharmaceutical composition of the present invention may further contain other pharmacological ingredients as far as they do not contradict the purpose of the present invention.

Further details of the present invention will be described below with reference to examples.

### Example 1

### (Method)

### Experimental animal surgeries

Six to eight-week-old Yorkshire crossbred pigs of either sex were housed individually, and maintained on a commercial pelleted feed. Pigs were held off feed for 24 hours prior to experimental surgery. General anesthesia was induced with xylazine (1.5 mg/kg, IM), ketamine (11 mg/kg, IM), and thiopental (15mg/kg, IV) and was maintained with intermittent infusion of thiopental (6 - 8mg/kg/hr) . Pigs were placed on a heating pad and ventilated with 100% O₂ via a tracheotomy using a time-cycled ventilator. The jugular vein and carotid artery were cannulated and blood gas analysis was performed to confirm normal pH and partial pressures of CO₂ and O₂. Lactated Ringers solution was administered intravenously at a maintenance rate of 15ml/kg/hr. The ileum was approached via ventral midline incision. Ileal segments were delineated by ligating the intestine at 10 cm intervals, and subjected to ischemia by occluding the local mesenteric blood supply for 45 minutes.

### Ussing chamber studies

Following the 45-minute ischemic period, tissues were harvested from the pig and the mucosa was stripped from the seromuscular layer in oxygenated (95% O₂/ 5% CO₂) Ringer's solution (mmol/l: Na⁺, 154; K⁺, 6.3; Cl⁻, 137; HCO₃⁻, 24; pH 7.4) containing 5x10⁻⁶ M indomethacin to prevent endogenous PG production during the stripping procedure. Tissues were then mounted in 3.14 cm² aperture Ussing chambers. For Ussing chamber experiments, ileal tissues from one pig were mounted on multiple Ussing chambers and subjected to different *in vitro* treatments. Tissues were bathed on the serosal and mucosal sides with 10ml Ringer's solution. The serosal bathing solution contained 10mM glucose, and was osmotically balanced on the mucosal side with 10mM mannitol. Bathing solutions were oxygenated (95% O₂/5% CO₂) and circulated in water-jacketed reservoirs. The spontaneous potential difference (PD) was measured using Ringer-agar bridges connected to colonel electrodes, and the PD was short-circuited through Ag-AgCl electrodes using a voltage clamp that corrected for fluid resistance. Transepithelial electrical resistance (Ω.cm²) was calculated from the spontaneous PD and short-circuit current (*I*_{sc}). If the spontaneous PD was between -1.0 and 1.0 mV, tissues were current clamped at ±100 µA for 5 seconds and the PD recorded. Short-circuit current and PD were recorded at 15-minute intervals over a 4-hour experiment.

### Experimental treatments

After tissues were mounted on Ussing chambers, tissues were allowed to acclimate for a period of 30 minutes to achieve stable baseline measurements. Tissues were then treated with varying doses of COMPOUND A (13,14-dihydro-15-keto-16,16-difluoro-PGE₁) (0.01µM, 0.1µM, and 1µM) by adding the compound to the mucosal bathing solution (t = 30 min).

### ³H-mannitol flux studies

These studies were performed at the same time as electrical measurements were recorded. To assess mucosal-to-serosal flux, ³H-mannitol was added to the mucosal solutions. Following a 15-minute equilibration period, standards were taken from the bathing reservoirs. Thirty minutes after the addition of treatments, three successive 60 minute flux periods (from 30 to 210 minutes of the experiments) were performed by taking samples from the bathing reservoirs opposite to the side of isotope addition. Samples were counted for ³H-mannitol in a liquid scintillation counter. Unidirectional mucosa-to-serosa (Jₘₛ) flux was determined using standard equations.

### Histological examination

Tissues were taken at 0, 60, and 180 minutes for routine histologic evaluation. Tissues were sectioned (5 µm) and stained with hematoxylin and eosin. For each tissue, 3 sections were evaluated. Four well oriented villi and crypts were identified in each section. Villus length was obtained using a micrometer in the eye piece of a light microscope. In addition, the height of the epithelial-covered portion of each villus was measured. The surface area of the villus was calculated using the formula for the surface area of a cylinder. The formula was modified by subtracting the area of the base of the villus, and multiplying by a factor accounting for the variable position at which each villus was cross-sectioned (Gastroenterology 1993; 104:440-471). The percentage of the villous surface area that remained denuded was calculated from the total surface area of the villus and the surface area of the villus covered by epithelium. The percent denuded villous surface area was used as an index of epithelial restitution.

### Statistical analysis

Data were reported as means ± SE. All data were analyzed by using an ANOVA for repeated measures, except where the peak response was analyzed by using a standard one-way ANOVA (Sigmastat, Jandel Scientific, San Rafael, CA). A Tukey's test was used to determine differences between treatments following ANOVA.

### (Results)

### The effect on short circuit current and transepithelial resistance across ischemia-injured porcine ileum

Porcine ileum was subjected to a 45-minute period of mesenteric ischemia and then mounted on Ussing chambers upon which short circuit current (I_{sc}), an indicator of Cl⁻ secretion, and transepithelial resistance (TER), an indicator of mucosal barrier function, were assessed. Forty five minutes of intestinal ischemia resulted in a 40% drop in TER compared with non-ischemic control tissue indicating that barrier function was impaired in the ischemic tissue. Application of 0.01µM, 0.1µM, and 1µM COMPOUND A to the mucosal side of ischemia-injured mucosa (Figure 1) induced dose-dependent increases in TER, with 1µM COMPOUND A stimulating a 2-fold increase in TER (ΔTER =26 Ω.cm², P<0.01) .

Application of 0.1 µM and 1 µM COMPOUND A to ischemic mucosa stimulated sharp and significant (P<0.05) peaks in I_{sc}, in a dose-dependant manner, indicating activation of electrogenic Cl⁻ secretion in these tissues. Similar dose responses were observed when assessing the effect of COMPOUND A on the absolute change in I_{sc} (Δ I_{sc} = 29±5.1, 16±4.5, and 2±0.8 for 1µM, 0.1µM, and 0.01µM COMPOUND A). Elevations in I_{sc} preceded increases in TER.

### The effect on mucosal to serosal flux of mannitol in the ischemia-injured porcine ileum.

Because mucosal to serosal flux of ³H-mannitol has been shown to be a sensitive indicator of mucosal barrier function, we measured ³H-mannitol flux across ischemia-injured mucosa to confirm TER values. Ischemic injury resulted in a significantly increased flux of mannitol compared with non-injured control tissue (Fig 3) indicating that barrier function was compromised in ischemic tissue. Application of 1 µM COMPOUND A resulted in the return of ³H-mannitol flux to non-ischemic control levels.

### Histological evaluation of ischemic tissue treated with COMPOUND A

Acute restoration of barrier function in injured mucosa involves 3 concerted mechanisms: (1) villus contraction which reduces the total denuded surface area for repair, (2) restitution or cell migration to seal the exposed basement membrane, and (3) closure of the paracellular space and tight junctions. To determine whether improvements in barrier function in response to COMPOUND A treatment were in part due to enhanced epithelial restitution, we evaluated histology of recovering ischemic tissues at several timepoints during the recovery period. Histological analysis of injured tissues revealed sloughing and lifting of the intestinal epithelium on the apical 1/3 of villi. This correlated to a 30% denuded surface area of the epithelium by morphometric analysis (Table 1). Within 60 minutes of mounting tissues on Ussing chambers, the intestinal villi had undergone rapid and complete restitution.

**Table 1. Morphometric assessment of epithelial restitution in ischemic-injured porcine ileal mucosa**

| **Treatment** | **Recovery time (min)** | **Epithelial surface area denuded (%)** | **Villus height (mm)** |
|---|---|---|---|
| Non-ischemic control | 0 | 0 ± 0 | 0.16 ± 0.02* |
| Ischemic | 0 | 30.2 ± 4.7 | 0.10 ± 0.01 |
| Ischemic/Indo | 60 | 6. 6 ± 2.6* | 0.16 ± 0.01* |
| Ischemic/Indo/COMPOUND A | 60 | 4.2 ± 2.5* | 0.21 ± 0.02* |
| Ischemic/Indo | 180 | 0 ± 0** | 0.14 ± 0.03* |
| Ischemic/Indo/COMPOUND A | 180 | 0 ± 0** | 0.20 ± 0.01* |

In table 1, values represents means ± SE for % villus surface area denuded and Villus height; n=3. Tissues were mammalian ileum subjected to 45 min ischemia in vivo, after which they were mounted in Ussing chambers for monitoring of recovery responses. Tissues were harvested at 0 min, 60 min, and 180 min post-ischemia during the in vitro recovery phase, fixed in 10% buffered formalin, and processed for histological examination according to standard protocols. Indomethacin (Indo) was administered to select tissues at 5µM, and COMPOUND A was given at 1µM. Values lacking common superscript (*,#) differ by P<0.05.

### Example 2

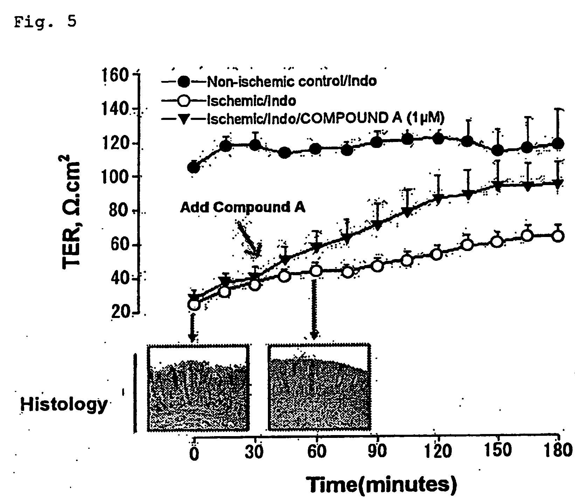

According to the same procedure described in Example 1 except for using colon instead of ileum, recovery of mucosal barrier function in ischemic condition by the COMPOUND A was investigated.
A) Change in short circuit current response to COMPOUND A in ischemia-injured porcine ascending colon, B) Trans epithelial electrical resistance (TER) in response to COMPOUND A in ischemia-injured porcine ascending colon and C) Serosal to mucosal ³H-mannitol fluxes in response to COMPOUND A in ischemia-injured porcine ascending colon were shown in figures 4 to 6 respectively.

Application of COMPOUND A to ischemic porcine ascending colon increased Isc(Figure 4) and TER and recuded serosal-to mucosal fluxes of ³H-mannitol (Figures 5 and 6).

### Conclusions

The data demonstrates that the ClC-2 agonist, COMPOUND A, stimulates Cl⁻ secretion and subsequent recovery of mucosal barrier function in ischemia-injured porcine ileum and colon. Further, the salutary effect of COMPOUND A on mucosal barrier function appears to be mediated through reductions in paracellular permeability and independent of epithelial restitution. These observations indicate that the ClC-2 agonist, COMPOUND A induces a conformational change in the tight junction that results in recovery of barrier function. Selective agonists of ClC-2 may provide a novel pharmacological means of hastening recovery of acutely injured intestine.

### Example 3

Female Crl:CD(SD)IGS BR VAF/Plus rats were assigned to 4 study groups (65/group). Groups 2 through 4 received 20, 100, or 400 µg/kg/day of COMPOUND A, respectively, by oral gavage for 104 weeks. The control group (Group 1) received the vehicle, a 1% aqueous solution of Polysorbate 80. The dose volume was 5 mL/kg/day for all groups. When unscheduled death of animal occurred during the study period, a necropsy was performed on the animal. After 104 weeks of treatment, all surviving animals were sacrificed and necropsied. Each rat was evaluated microscopically for the occurrence of mammary carcinoma.

As shown in Table 2, COMPOUND A reduced the incidence of mammary carcinoma.

**Table 2. Incidence of mammary carcinoma**

| Group | Dose µg/kg/day | Number of animals examined | Number of animals with mammary carcinoma |
|---|---|---|---|
| 1. Control (Vehicle) | 0 | 65 | 12 |
| 2. COMPOUND A | 20 | 65 | 6 |
| 3. COMPOUND A | 100 | 65 | 5 |
| 4. COMPOUND A | 400 | 63 | 4 |

## Claims

1. Use of 13,14-dihydro-15-keto-16,16-difluoro-prostaglandin E₁ or 13,14-dihydro-15-keto-16,16-difluoro-18-methyl-prostaglandin E₁ for manufacturing a pharmaceutical composition for treating cancer or premalignant condition in a mammalian subject.

2. The use as described in Claim 1, wherein said cancer is selected from the group consisting of esophageal carcinoma, gastric carcinoma, duodenal cancer, small intestinal cancer, appendiceal cancer, large bowel cancer, colon cancer, rectum cancer, anal carcinoma, pancreatic cancer, liver cancer, gallbladder cancer, spleen cancer, renal carcinoma, bladder cancer, prostatic carcinoma, testicular carcinoma, uterine cancer, ovarian cancer, mammary carcinoma, pulmonary carcinoma and thyroid carcinoma.

3. A composition for use in the treatment of cancer or premalignant condition in a mammalian subject, which comprises an effective amount of 13,14-dihydro-15-keto-16,16-difluoro-prostaglandin E₁ or 13,14-dihydro-15-keto-16,16-difluoro-18-methyl-prostaglandin E₁.

4. The composition for use as described in Claim 3, wherein said cancer is selected from the group consisting of esophageal carcinoma, gastric carcinoma, duodenal cancer, small intestinal cancer, appendiceal cancer, large bowel cancer, colon cancer, rectum cancer, anal carcinoma, pancreatic cancer, liver cancer, gallbladder cancer, spleen cancer, renal carcinoma, bladder cancer, prostatic carcinoma, testicular carcinoma, uterine cancer, ovarian cancer, mammary carcinoma, pulmonary carcinoma and thyroid carcinoma.

## Patentansprüche

1. Verwendung von 13,14-Dihydro-15-keto-16,16-difluor-prostaglandin E₁ oder 13,14-Dihydro-15-keto-16,16-difluor-18-methyl-prostaglandin E₁ zur Herstellung einer pharmazeutischen Zusammensetzung zur Behandlung von Krebs oder einem prämalignen Zustand bei einem Säugetiersubjekt.

2. Verwendung, wie sie in Anspruch 1 beschrieben ist, wobei der Krebs aus der Gruppe, bestehend aus ösophagealem Karzinom, Magenkarzinom, Duodenalkarzinom, Dünndarmkrebs, Appendixkrebs, Dickdarmkrebs, Kolonkrebs, Rektumkrebs, Analkarzinom, Pankreaskrebs, Leberkrebs, Gallenblasenkrebs, Milzkrebs, Nierenkarzinom, Blasenkrebs, Prostatakarzinom, Hodenkarzinom, Uteruskrebs, Eierstockkrebs, Brustkrebs, Lungenkarzinom und Schilddrüsenkarzinom, ausgewählt ist.

3. Zusammensetzung zur Verwendung bei der Behandlung von Krebs oder einem prämalignen Zustand bei einem Säugetiersubjekt, die eine wirksame Menge von 13,14-Dihydro-15-keto-16,16-difluor-prostaglandin E₁ oder 13,14-Dihydro-15-keto-16,16-difluor-18-methyl-prostaglandin E₁ umfasst.

4. Zusammensetzung zur Verwendung, wie sie in Anspruch 3 beschrieben ist, wobei der Krebs aus der Gruppe, bestehend aus ösophagealem Karzinom, Magenkarzinom, Duodenalkarzinom, Dünndarmkrebs, Appendixkrebs, Dickdarmkrebs, Kolonkrebs, Rektumkrebs, Analkarzinom, Pankreaskrebs, Leberkrebs, Gallenblasenkrebs, Milzkrebs, Nierenkarzinom, Blasenkrebs, Prostatakarzinom, Hodenkarzinom, Uteruskrebs, Eierstockkrebs, Brustkrebs, Lungenkarzinom und Schilddrüsenkarzinom, ausgewählt ist.

## Revendications

1. Utilisation de 13,14-dihydro-15-céto-16,16-difluoro-prostaglandine E₁ ou de 13,14-dihydro-15-céto-16,16-difluoro-18-méthyl-prostaglandine E₁ pour la fabrication d'une composition pharmaceutique destinée au traitement du cancer ou d'une affection prémaligne chez un sujet mammifère.

2. Utilisation telle que décrite dans la revendication 1, où ledit cancer est choisi dans le groupe consistant en carcinome oesophagien, carcinome gastrique, cancer duodénal, cancer de l'intestin grêle, cancer de l'appendice, cancer du gros intestin, cancer du côlon, cancer du rectum, carcinome anal, cancer pancréatique, cancer du foie, cancer de la vésicule biliaire, cancer de la rate, carcinome rénal, cancer de la vessie, carcinome prostatique, carcinome testiculaire, cancer de l'utérus, cancer ovarien, carcinome mammaire, carcinome pulmonaire et carcinome de la thyroïde.

3. Composition pour une utilisation dans le traitement du cancer ou d'une affection prémaligne chez un sujet mammifère, qui comprend une quantité efficace de 13,14-dihydro-15-céto-16,16-difluoro-prostaglandine E₁ ou de 13,14-dihydro-15-céto-16,16-difluoro-18-méthyl-prostaglandine E₁.

4. Composition pour une utilisation telle que décrite dans la revendication 3, où ledit cancer est choisi dans le groupe consistant en carcinome oesophagien, carcinome gastrique, cancer duodénal, cancer de l'intestin grêle, cancer de l'appendice, cancer du gros intestin, cancer du côlon, cancer du rectum, carcinome anal, cancer pancréatique, cancer du foie, cancer de la vésicule biliaire, cancer de la rate, carcinome rénal, cancer de la vessie, carcinome prostatique, carcinome testiculaire, cancer de l'utérus, cancer ovarien, carcinome mammaire, carcinome pulmonaire et carcinome de la thyroïde.
